# EUROPEAN PATENT SPECIFICATION

(11) **EP 1 980 248 B1**
(45) Date of publication and mention of the grant of the patent: **12.06.2019**
(21) Application number: 08006865.3
(22) Date of filing: 04.04.2008
(51) Int. Cl.: A61K 31/352, C07D 311/30, A61P 35/02

(54) **Composition for treating cancer cells and synthetic method for the same**
Zusammensetzung zur Behandlung von Krebszellen und synthetisches Verfahren dafür
Composition pour le traitement des cellules cancéreuses et son procédé synthétique

(30) Priority: 10.04.2007 TW 96112618
(43) Date of publication of application: 15.10.2008
(73) Proprietor: Kaohsiung Medical University, 807 Kaohsiung City (TW)
(72) Inventor: An-Shen, Lin, Kaohsiung City 807 (TW); Yang-Chang, Wu, Kaohsiung City 807 (TW); Kuo-Hsiung, Lee, Kaohsiung City 807 (TW); Fang-Rong, Chang, Kaohsiung City 807 (TW)
(74) Representative: Bittner, Thomas L.

(56) References cited:
- US-A1- 2007 104 804
- US-B1- 6 340 694
- LIN AN-SHEN ET AL: "New cytotoxic flavonoids from Thelypteris torresiana." PLANTA MEDICA SEP 2005, vol. 71, no. 9, September 2005 (2005-09), pages 867-870, XP002491003 ISSN: 0032-0943
- FELPIN ET AL: "Oxidation of 4-arylphenol trimethylsilyl ethers to p-arylquinols using hypervalent iodine(III) reagents" TETRAHEDRON LETTERS, ELSEVIER, AMSTERDAM, vol. 48, no. 3, 14 December 2006 (2006-12-14), pages 409-412, XP005730747 ISSN: 0040-4039
- LIN AN-SHEN ET AL: "First total synthesis of protoapigenone and its analogues as potent cytotoxic agents." JOURNAL OF MEDICINAL CHEMISTRY 9 AUG 2007, vol. 50, no. 16, 9 August 2007 (2007-08-09), pages 3921-3927, XP002491004 ISSN: 0022-2623
- CHANG HSUEH-LING ET AL: "Protoapigenone, a novel flavonoid, induces apoptosis in human prostate cancer cells through activation of p38 mitogen-activated protein kinase and c-Jun NH2-terminal kinase 1/2" JOURNAL OF PHARMACOLOGY AND EXPERIMENTAL THERAPEUTICS, EXPERIMENTAL THERAPEUTICS, BALTIMORE, MD, vol. 325, no. 3, 1 June 2008 (2008-06-01), pages 841-849, XP009104206 ISSN: 0022-3565
- SHAH M ET AL: "Antitubercular properties of substituted hydroxycyclohexadienones" LETTERS IN DRUG DESIGN AND DISCOVERY, BENTHAM SCIENCE PUBLISHERS, US, vol. 3, no. 6, 1 January 2006 (2006-01-01), pages 419-423, XP001538528 ISSN: 1570-1808
- WADA H ET AL: "CHEMICAL AND CHEMOTAXONOMICAL STUDIES OF FERNS LXXIII. NEW FLAVONOIDS WITH MODIFIED B-RING FROM THE GENUS PSEUDOPHEGOPTERIS THELYPTERIDACEAE", CHEMICAL AND PHARMACEUTICAL BULLETIN, PHARMACEUTICAL SOCIETY OF JAPAN, JP, vol. 35, no. 12, 1 January 1987 (1987-01-01), pages 4757-4762, XP001538526, ISSN: 0009-2363

## Description

The present invention relates to a composition for treating cancer cells and describes a synthetic method therefor. More particularly, the method is total synthesis or semi-synthesis.

Recently many extracts and their derivatives oriented from natural plants, such as vincristine, vinbalstine, camptothecin, taxol and its derivatives, palitaxel and docetaxel, have been widely used in clinical chemical therapy of a malignant tumor. Therefore, the effect of the extracts from natural plants has been a burgeoning research in the field of new drug development.

According to present publication, protoapigenone is a compound derived from *Thelypteris torresiana* produced in Taiwan. The compound has strong cytotoxic activities to many human cancer cell lines, including breast cancer cells (MCF-7, MDA-MB-231), liver cancer cells (Hep G2, and Hep 3B), and lung cancer cells (A549). (Lin et al, Planta Medica 71:867-870, 2005)

Prior to that, the protoapigenone can be obtained merely by extraction and isolation from natural plants. However, the efficiency of collecting the compound is under influence of unstable sources such as plant genes, humidity, and latitude, altitude of the collection location, season and individual variation of the plants.
Felpin (Tetrahedron Lett. 48:409-412, 2007) describes the use of hypervalent iodine(III) reagents for an oxidation of phenol derivatives, and the use of TEMPO as catalyst. Also Shah et al. (Lett. Drug Design & Discov. 3:419-423, 2006) describe the hypervalent iodine(III) mediated oxidation of phenol derivatives.
US 6,340,694 B1 describes diarylbenzopyran derivatives and their chemical synthesis.
Wada et al. (Chem. Pharm. Bull 35:4757-4762, 1987) discloses flavonoids with modified B-ring from the Genus Pseudophegopteris.

In view of the above, the inventors develop a method for producing a compound for treating cancer cells through total synthesis or semi-synthesis based on the inventors' experience in studying the ingredient of natural plants and chemical synthesis for a long time. By using such preparation, the protoapigenone can be produced from commercially available chemical products without extracting from natural plants. Moreover, the activity of the synthetic compound has no obvious difference with nature product through a comparison of activity. Moreover, the source of the compound would be stable and preparation time would be saved. Further, yield of the compound can be estimated in a large scale production by using the synthetic method described herein.

Besides, many derivatives of protoapigenone will be produced from different initiators, reagents and intermediator. Moreover, the acidity, the alkalinity, the lipid solubility, the solubility, the activity and the toxicity of the compound can be changed by substituting with different functional groups so that a more potent compound with cytotoxic effect can be afforded. The summary of the present invention is described below.

It is an aspect of the present invention to provide a pharmaceutical composition having a cytotoxic effect to a cancer cell. The pharmaceutical composition comprises a flavonoid compound having the following formula 2: 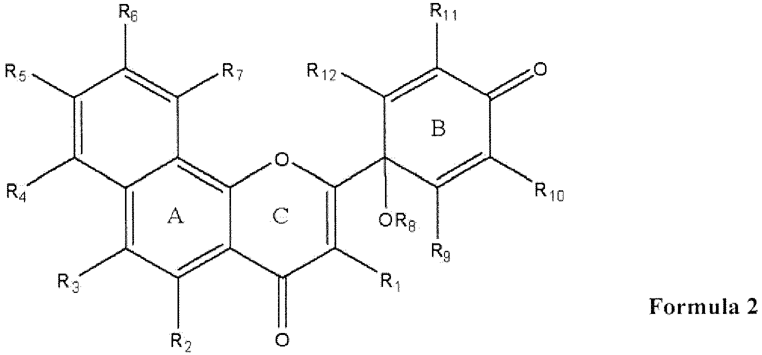 wherein B ring is a 4-oxo-cyclohexa-2,5-dienyl group, and any one of R₁-R₁₂ is one selected from a group consisting of hydrogen group, hydroxyl group, C1-C20 alkyl group, C1-C20 ether group, C1-C20 ester group, carboxyl group, halogen and sugar. In one preferred embodiment of this invention, flavonoid compound is obtained from a total synthesis method. In another preferred embodiment, the flavonoid compound is obtained from a semi-synthesis method.

Preferably, the pharmaceutical composition is used for treating a disease of a mammal.

Preferably, the disease is a cancer.

Preferably, the mammal is a human.

Also described are pharmaceutical compositions comprising a flavonoid compound having one of the following formulas 1 and 3: 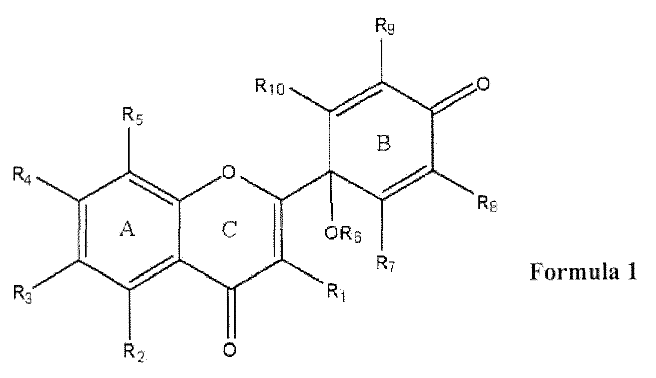 and

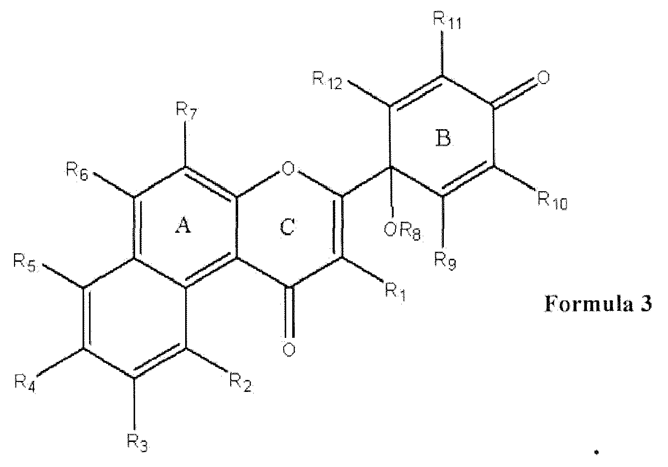

Herein, a method for synthesizing a specific flavonoid compound is described. The method comprises steps of mixing an acetophenone comprising a first protecting group with a benzaldehyde comprising a second protecting group to obtain a first compound through a Claisen-Schmidt Condensation reaction, reacting the first compound with a first catalyzer to obtain a second compound, removing the second protecting group from the second compound to obtain a third compound, reacting the third compound with a second catalyzer and an iodobenzene compound to obtain a fourth compound through an oxidation, and adding an acid into the fourth compound to remove the first protecting group and obtain the flavonoid compound. For example, the specific flavonoid compound is protoapigenone.

For example, the first protecting group is a MOM.

For example, the second protecting group is a benzyloxy group.

For example, the first catalyzer is an iodine.

For example, the second catalyzer is a TEMPO.

For example, the iodobenzene compound is one of an iodobenzene diacetate and a [bis(trifluoroacetoxy)iodo]benzene.

For example, the acid is hydrochloric acid.

A further method for synthesizing a specific flavonoid compound is described herein. The method comprises steps of providing a flavonoid compound comprising a 4'-hydroxy group, and mixing the flavonoid compound with a first catalyzer and an iodobenzene compound to obtain the specific flavonoid compound. In an exemplary embodiment, the specific flavonoid compound is derivatives of protoapigenone.

For example, the first catalyzer is a TEMPO.

For example, the iodobenzene compound is one of an iodobenzene diacetate and an [bis(trifluoroacetoxy)iodo]benzene.

Other objects, advantages and efficacies of the present invention will be described in detail below taken from the preferred embodiments with reference to the accompanying drawings, in which:
Fig. 1 is a flow chart showing the total synthesis method for preparing the flavonoid compound of Formula 1 described in the present invention;
Fig. 2 shows chemical structures of compounds 8a, 10a, 15a and 18a, i.e. compounds of Formula 1 or 3, prepared by a semi-synthesis method in the present invention; and
Fig. 3 shows chemical structures of compounds 8b, 10b, and 15b, i.e. compounds of Formula 1 or 3, prepared by a semi-synthesis method in the present invention.

### I. Definitions

The following definitions are provided in order to aid understanding of the detailed description of the present invention:
As used herein, the term "C1-C20 alkyl" represents a straight- or branched-chain saturated hydrocarbon containing 1 to 20 carbon atoms which may be unsubstituted or substituted by one or more substituents. Examples of C1-C20 alkyl groups include methyl, ethyl, propyl, 2-propyl, n-butyl, iso-butyl, tert-butyl, pentyl, and the like. Similarly, the term "C1-C20 ether" and "C1-C20 ester" represents a compound containing 1 to 20 carbon atoms which may be unsubstituted or substituted by one or more substituents.

As used herein, the term "alkyl" represents both straight and branched, saturated and unsaturated hydrocarbon groups.

As used herein, the term "ether" represents any of a number of organic compounds whose molecules contain two hydrocarbon groups joined by single bonds to an oxygen atom.

As used herein, the term "ester" refers to and covers any compound falling within the definition of that term as classically used in organic chemistry. It includes organic and inorganic esters, where A is -COOH; this term covers the products derived from treatment of the function with alcohols or thioalcohols. The ester is derived from compounds where A is --CH₂ OH; this term covers compounds derived from organic acid capable of forming esters such as phosphorous-based and sulfur-based acids, or compounds of the formula --CH₂ OCOR where R is any substituted or unsubstituted aliphatic aromatic, heteroaromatic or aliphatic-aromatic group.

The term "halogen" refers to fluorine, chlorine, bromine or iodine.

The term "total synthesis" refers to the chemically synthetic method using starting materials other than one of the naturally occurring compounds. The term "semi-synthesis" refers to the chemical synthetic method using naturally occurring compounds as starting materials.

As used herein, the term "mammal" refers to the Mammalia class of higher vertebrates, especially human.

The term "protecting" as used herein refers to a process in which a functional group in a chemical compound is selectively masked by a non-reactive functional group in order to allow a selective reaction(s) to occur elsewhere on the chemical compound. Such non-reactive functional groups are herein termed as "protecting groups." Such groups are generally able to be selectively introduced and removed using mild reaction conditions that do not interfere with other portions of the subject compounds.

The term "oxidation" refers to the loss of one or more electrons in an element, compound, or chemical substituent/subunit. In an oxidation reaction, electrons are lost by atoms of the element(s) involved in the reaction. The charge on these atoms must then become more positive. The electrons are lost from the species undergoing oxidation and thus electrons appear as products in an oxidation reaction.

The term "protoapigenone" used in the embodiment refers to a flavonoid compound containing a 4'-oxocyclohexa-2',5'-dienyl moiety rather than a 4'-hydroxycyclohexa-2',5'-dienyl moiety in a nature product. Except the particularity mentioned "natural protoapigenone" or "protoapigenone extracted from a plant", the term "protoapigenone" mentioned in the embodiment represents the flavonoid derived from a chemical synthesis method.

### II. Detailed Description

### Example I: The total synthetic method for preparing protoapigenone compound (a compound of Formula 1)

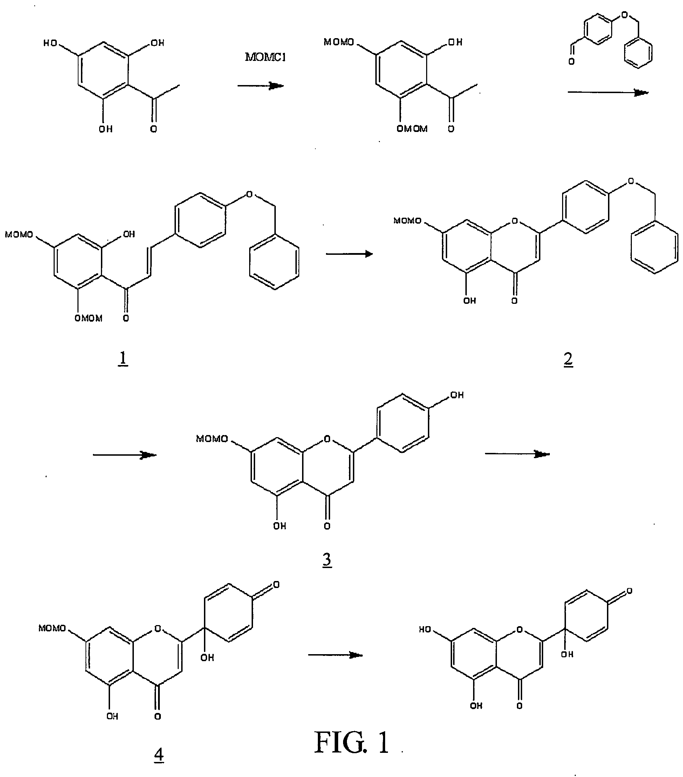

Please refer to Fig. 1, which is a flowchart showing a total synthesis method for preparing the flavonoid compound in the present invention. Firstly, an exceed amount (for example, 7E (equivalent)) of potassium carbonate is added into an acetone solution containing 2',4',6'-Trihydroxy-acetophenone monohydrate to form a first mixture, and the first mixture is stirred. Then, 3E of chloromethyl methyl ether is added dropwise into the first mixture. For fully reacting, the first mixture is refluxed for 90 minutes. After cooling, the first mixture is filtrated to obtain a precipitation. The precipitation is rinsed with acetone and chloroform, and then evaporated and purified by column chromatography on a silica gel (isocratic elution, 90% *n*-hexane / 10% ethyl acetate) to obtain a 2'-hydroxy-4'-6'-dimethoxymethyl-acetophenone with MOM protecting groups on the 4' and the 6' positions in 50.3% yield.

Subsequently, the aforementioned 2'-hydroxy-4'-6'-dimethoxymethyl-acetophenone and a 4-benzyloxy-benaldehyde are mixed to carry out a Claisen-Schmidt Condensation reaction. In this condensation reaction, 2'-hydroxy-4'-6'-dimethoxymethyl-acetophenone and 2E of 4-benzyloxy-benaldehyde are mixed in ethanol solution to obtain a second mixture. After stirring the second mixture, a catalytic amount of potassium hydroxide is added thereinto. The second mixture is stirred at room temperature for 30 hrs, and then the solvent was evaporated under reduced pressure. The concentrated second mixture is chromatographed on a silica gel and eluted with n-hexane/ethyl acetate to obtain a first compound in 87.2% yield.

0.2E of iodine is added into a proper amount of pyridine solution containing the dissolved first compound. After the pyridine solution is heated and refluxed for 5 hrs, a sodium thiosulfate is added thereinto. The pyridine solution is extracted with ethyl acetate/water and then purified by a column chromatography on silica gel to obtain a second compound in 85.5% yield.

Then, the second compound is dissolved in an ethyl acetate/methanol solution. 1E of 10% palladium carbon (Pd/C) is added into the ethyl acetate/methanol solution and the solution mixture is stirred under an atmosphere of hydrogen for 3 hrs. After a filtration process for removing the palladium carbon, the above solution is concentrated under reduced pressure to obtain a third compound in 88.9% yield.

An oxidation reaction is carried out by reacting the third compound with 1E of iodobenzene diacetate or [bis(trifluoroacetoxy)iodo]benzene in the presence of a catalytic amount of TEMPO under a heating condition to obtain a reactant a. The reactant a is extracted with ethyl acetate/water and then isolated repeatedly on a silica gel column to obtain a forth compound in 22.2% yield.

The MOM protecting groups on the 4' and the 6' positions of the forth compound is removed by heating with hydrochloric acid to provide a protoapigenone compound in 46.7% yield.

### Example II: The semi-synthesis method for preparing derivatives of protoapigenone (compounds of Formula 1 or 3)

The embodiment herein illustrates a semi-synthesis method for synthesizing the following compounds: compound 8a: protoflavonone (IIUPAC name is 2-(1-hydroxy-4-oxocyclohexa-2,5-dienyl)-4H-chromen-4-one), compound 10a: 5-hydroxyprotoflavone (IIPAC name is 2-(1-hydroxy-4-oxocyclohexa-2,5-dienyl)-5-hydroxy-4H-chromen-4-one), compound 15a: β-nanphthoflavonone (IIPAC name is 3-(1-hydroxy-4-oxocyclohexa-2,5-dienyl)-1H-benzo[f]chromen-1-one) and compound 18a: 5-hydroxy-7-methoxyprotoflavonone (IIPAC name is 5-hydroxy-2-(1-hydroxy-4-oxocyclohexa-2,5-dienyl)-7-methoxy-4H-chromen-4-one).

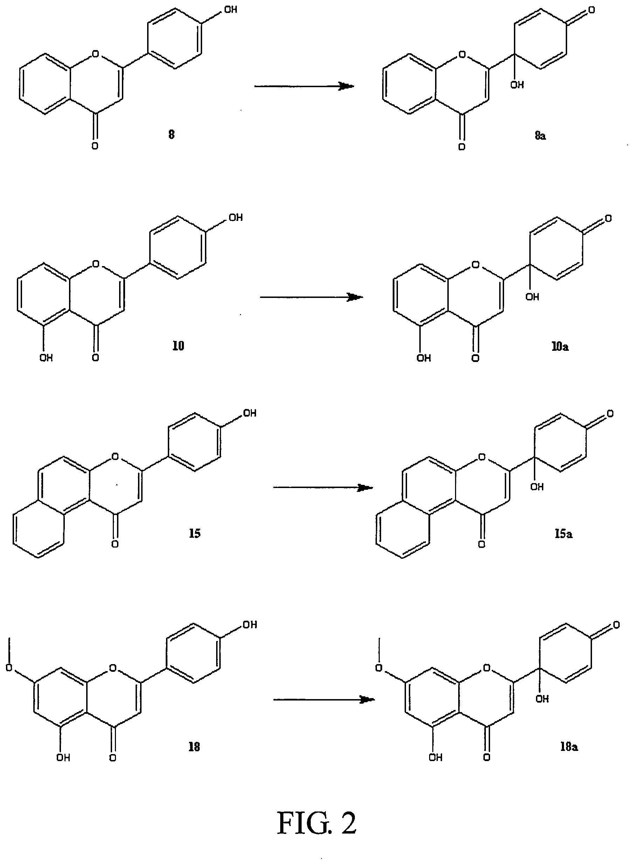

Firstly, a commercially available precursor flavonoid such as 4'-hydroxyflavone, 4'-5-dihydroxyflavone, 4-hydroxy-β-naphthoflavone and 4'-5-dihydroxy-7-methoxyflavone is selected as a starting material and dissolved in an appropriate amount of water. Secondly, a catalytic amount of TEMPO is added to the precursor flavonoid. The solution mixture is oxidized with 1E of iodobenzene diacetate or [bis(trifluoroacetoxy)iodo]benzene in a heating condition to obtain a product b. Sequentially, the product b is extracted with ethyl acetate/water to afford an organic layer, and the organic layer is isolated by chromatography repeatedly to obtain the compound 8a, compound 10 a, compound 15a and compound 18a in 20-30% yield. The structural changes of these compounds are shown in Fig. 2.

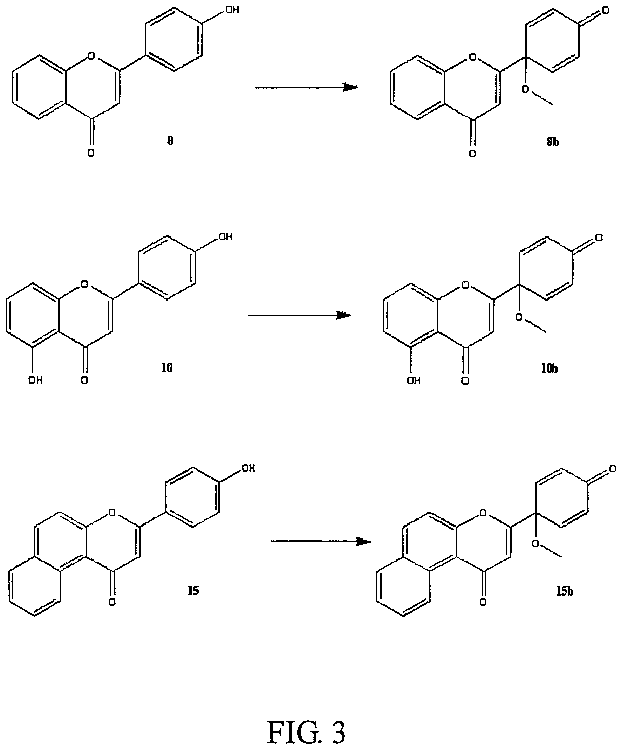

In another way, the above precursor flavonoids can be dissolved in an appropriate amount of methanol. A catalytic amount of TEMPO is added to the precursor flavonoid respectively. The solution mixture is oxidized with 1E of iodobenzene diacetate or [bis(trifluoroacetoxy)iodo]benzene in a heating condition to obtain a product c. Similarly, the product c is extracted with ethyl acetate/water to afford an organic layer, and the organic layer is isolated by chromatography repeatedly to obtain a compound having a methoxy group on the 1'-position such as 1'-methoxyprotoflavonoen (compound 8b), 1'-methoxy-5-methylprotoflavonone (compound 10 b), and 1'-methoxy-β-naphthoflavone (compound 15b) in 25-35% yield. The structural changes of these compounds are shown in Fig. 3.

### Example III: The bio-activities of the derivatives of protoapigenone (compounds of Formula 1 or 3)

### Cells culture

Human liver (HepG2 and Hep3B), breast (MCF-7 and MDA-MB-231), and lung (A549) cancer cell lines were propagated in RPMI-1640 medium supplement with 10% (v/v) FBS, 100 U/mL penicillin and 100 g/mL streptomycin at 37°C in a humidified atmosphere of 5% CO2 and 95% air. The culturing medium is changed every three days. Upon confluency, cells were subcultured to confluency, and used for experiments. The experimental period is determined according to the aim of experiment.

### Cytotoxicity assay

Cytotoxicity is measured by the MTT colorimetric method. Cells were seeded at densities of 5,000 - 10,000 cells/well in 96-well tissue culture plates. On day two, cells are treated with test compounds for another 72 hrs. After drug treatment, attached cells were incubated with MTT (0.5 mg/mL) for 1 hr and subsequently solubilized in DMSO. The absorbency at 550 nm is then measured using a microplate reader. The result of cell viability is shown in Table 1. The IC50 is the concentration of agent that reduces the cell viability by 50% under the experimental conditions, and the cytotoxic compound, Doxorubicin, is used as a positive control.

**Table 1**

| | **IC₅₀ (µg/mL)** | | | | |
|---|---|---|---|---|---|
| **Compound** | **HepG2** | **Hep3B** | **MDA-MB-231** | **MCF-7** | **A549** |
| **protoapigenone** | 2.32 | 0.65 | 0.41 | 1.07 | 3.96 |
| **8a** | 1.71 | 0.32 | 0.18 | 0.44 | 1.33 |
| **8b** | 10.95 | 1.45 | 1.47 | 2.14 | >20 |
| **10a** | 1.34 | 0.35 | 0.18 | 0.93 | 1.37 |
| **10b** | 7.64 | 0.66 | 0.54 | 1.55 | 16.36 |
| **15a** | 1.08 | 0.09 | 0.12 | 0.20 | 0.55 |
| **15b** | 3.16 | 0.35 | 0.37 | 0.86 | 8.20 |
| **18a** | 0.17 | 0.20 | 0.13 | 0.51 | 0.93 |
| **Doxorubicin** | 0.29 | 0.36 | 0.08 | 0.43 | 0.21 |

As shown in Table 1, each test compound is examined for *in vitro* cytotoxic activities against several human cancer lines and compared with the related flavonoid protoapigenone and doxorubicin, a positive control. Remarkably, compounds 8a, 10a, 15a and 18a show significant cytotoxic activity with notable IC₅₀ against cancer cell lines, especially Hep3B and MDA-MB-231. It is known that both the protoapigenone compound and its derivatives synthesized by a chemical method have cytotoxic effect against cancer cells, similar to nature protoapigenone compound extracted from a plant. Among the derivatives, compound 18a shows the highest potency against all five cancer cell lines. Amazingly, this compound also exhibits significantly enhanced activity against the HepG2 human liver cancer cell line, while most analogs show comparatively weak activity against this cell line. From the above data, it is proved that many derivatives having more potency to particular cancer cells than protoapigenone can be produced by using the synthetic method described in the present invention.

To summarize, the present invention provides a composition having a cytotoxic effect to a cancer cell and a method for synthesizing the same. The total synthesis or semi-synthesis method can generate the cytotoxic compound protoapigenone. Further, the method of the present disclosure can produce other derivatives with similar activity or even with better activity.

## Claims

1. A pharmaceutical composition having a cytotoxic effect on a cancer cell comprising:
a flavonoid compound having the following formula: 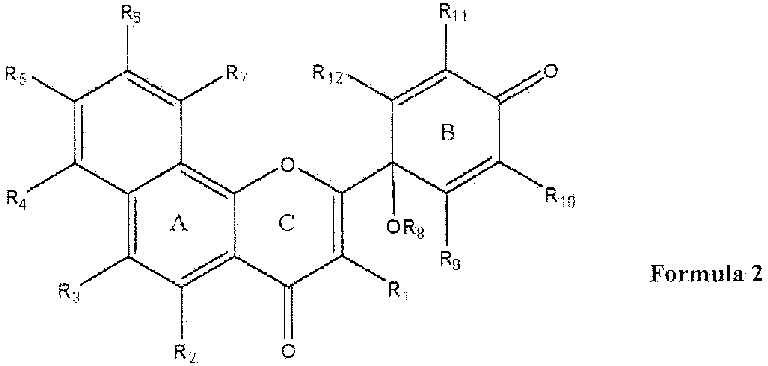 wherein B ring is a 4-oxo-cyclohexa-2,5-dienyl group, and any one of R1-R12 is one selected from a group consisting of hydrogen group, hydroxyl group, C1-C20 alkyl group, C1-C20 ether group, C1-C20 ester group, carboxyl group, halogen and sugar.

2. The composition as claimed in Claim 1, **characterized in that** the flavonoid compound is obtained from a chemical method being one of a total synthesis method and a semi-synthesis method.

3. The pharmaceutical composition of Claim 1 for use in the treatment of a disease of a mammal,
wherein the disease is a cancer.

4. The pharmaceutical composition for use according to Claim 3, **characterized in that** the mammal is a human.

## Patentansprüche

1. Pharmazeutische Zusammensetzung mit einer zytotoxischen Wirkung auf eine Krebszelle, umfassend:
eine Flavonoid-Verbindung mit der folgenden Formel: 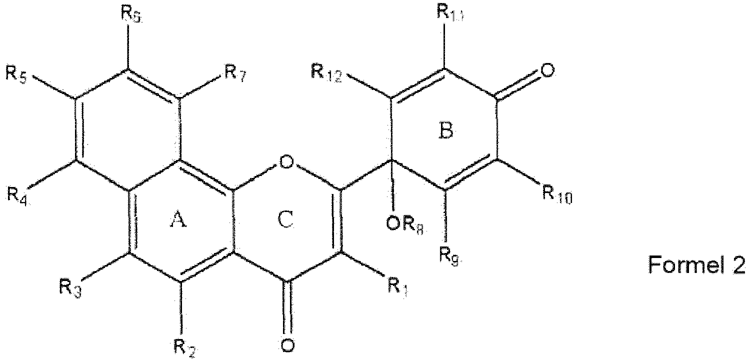 wobei Ring B eine 4-Oxo-cyclohexa-2,5-dienylgruppe ist und jedes von R1 bis R12 eines ist, das aus einer Gruppe ausgewählt ist, die aus einer Wasserstoffgruppe, einer Hydroxylgruppe, einer C1-C20-Alkylgruppe, einer C1-C20-Ethergruppe, einer C1-C20-Estergruppe, einer Carboxylgruppe, einem Halogen und einem Zucker besteht.

2. Zusammensetzung nach Anspruch 1, **dadurch gekennzeichnet, dass** die Flavonoid-Verbindung durch ein chemisches Verfahren erhalten wird, die eines aus einem Totalsyntheseverfahren und einem Halbsyntheseverfahren ist.

3. Pharmazeutische Zusammensetzung nach Anspruch 1 zur Verwendung bei der Behandlung einer Erkrankung eines Säugetiers,
wobei die Erkrankung ein Krebs ist.

4. Pharmazeutische Zusammensetzung zur Verwendung nach Anspruch 3, **dadurch gekennzeichnet, dass** das Säugetier ein Mensch ist.

## Revendications

1. Composition pharmaceutique ayant un effet cytotoxique sur une cellule cancéreuse comprenant :
un composé flavonoïde ayant la formule suivante : 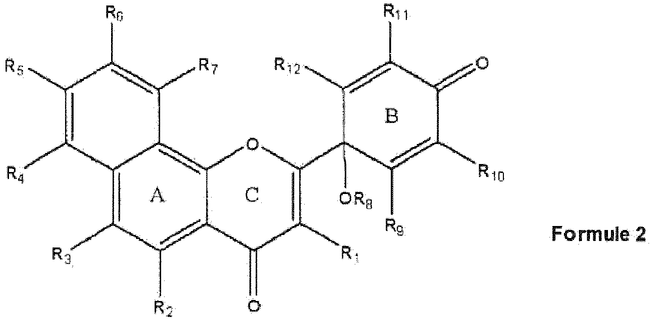 dans laquelle cycle B est un groupe 4-oxo-cyclohexa-2,5-diényle, et un quelconque de R1 à R12 est un sélectionné dans un groupe consistant en un groupe hydrogène, un groupe hydroxyle, un groupe alkyle en C1-C20, un groupe éther en C1-C20, un groupe ester en C1-C20, un groupe carboxyle, un halogène et un sucre.

2. Composition selon la revendication 1, **caractérisée en ce que** le composé flavonoïde est obtenu par un procédé chimique étant l'un d'un procédé de synthèse totale et d'un procédé de semi-synthèse.

3. Composition pharmaceutique selon la revendication 1 pour son utilisation dans le traitement d'une maladie d'un mammifère,
dans lequel la maladie est un cancer.

4. Composition pharmaceutique pour son utilisation selon la revendication 3, **caractérisée en ce que** le mammifère est un être humain.
